# EUROPÄISCHE PATENTSCHRIFT

(11) **EP 0 975 717 B1**
(45) Veröffentlichungstag und Bekanntmachung des Hinweises auf die Patenterteilung: **13.11.2002**
(21) Anmeldenummer: 98919233.1
(22) Anmeldetag: 08.04.1998
(51) Int. Cl.: C11C 3/00, C07C 303/06

(54) **VERFAHREN ZUR HERSTELLUNG VON SULFATIERTEN FETTSÄUREESTERN**
METHOD FOR PRODUCING SULFATED FATTY ACID ESTERS
PROCEDE DE PRODUCTION D'ESTERS D'ACIDE GRAS SULFATES

(30) Priorität: 16.04.1997 DE 19715833
(43) Veröffentlichungstag der Anmeldung: 02.02.2000
(73) Patentinhaber: Cognis Deutschland GmbH & Co. KG, 40589 Düsseldorf (DE)
(72) Erfinder: BIGORRA, Llosas, Joaquin, E-08203 Sabadell (ES); SEGURA, Ramon, E-08029 Barcelona (ES); PI SUBIRANA, Rafael, E-08400 Granollers (ES); FABRY, Bernd, D-41352 Korschenbroich (DE)
(86) Internationale Anmeldenummer: EP9802028
(87) Internationale Veröffentlichungsnummer: WO98046711

(56) Entgegenhaltungen:
- EP-A- 0 247 509
- EP-A- 0 327 938
- EP-A- 0 353 704

## Beschreibung

### Gebiet der Erfindung

Die Erfindung betrifft ein Verfahren zur Herstellung von Sulfatierungsprodukten von ungesättigten Fettsäureestern mit vermindertem Elektrolytgehalt durch Umsetzung der Ester mit Sulfatierungsmitteln, Überführung nicht abreagierter Schwefelsäure durch Ringöffnung von gegebenenfalls estergruppensubstituierter Epoxyalkanen in organisch gebundenes Sulfat und nachfolgende Neutralisation sowie die Verwendung der Verfahrensprodukte zur Herstellung von Mitteln für die Behandlung von Ledern und Pelzen.

### Stand der Technik

Neben den Gerbstoffen sind Fettungsmittel die wichtigsten Hilfsmittel, um den Charakter von Leder zu prägen. Die Wirkung der Fettungsmittel kommt durch eine faserisolierende Schmierung und durch eine Hydrophobierung zustande. Durch Umhüllung der Lederfasern mit einem Fettfilm wird die gegenseitige Reibung verringert und demzufolge die Geschmeidigkeit und Dehnbarkeit des Gewebes verbessert. Das hat positive Auswirkungen auf die Reißfestigkeit des Leders, denn in einem dehnbaren Werkstoff richten sich viele Fasern bei Zugbeanspruchung in der Zugrichtung aus und setzen dann dem Zerreißen einen größeren Widerstand entgegen als dieselben Fasern innerhalb eines spröden Werkstoffes. Durch die Hydrophobierung werden darüber hinaus gerbende Effekte erzielt, da sie mit einer Verdrängung von Wasser aus der Haut verbunden ist.

Als Lederfettungsmittel werden im allgemeinen pflanzliche und tierische Öle, Fette und Wachse eingesetzt, ferner die aus diesen Stoffen durch chemische Umwandlung gewonnenen Hydrolyse-, Sulfierungs-, Oxidations- und Härtungsprodukte und schließlich mineralische Fettungsmittel; im einzelnen:
- Die verseifbaren Fette und Öle sowie die natürlichen Wachse und Harze gehören zu den Estern. Unter Ölen und Fetten werden dabei vom Lederfachmann Ester aus Glycerin und Fettsäuren bezeichnet, die bei Raumtemperatur fest bzw. flüssig sind. Zur Lederfettung werden dabei aus der Gruppe der tierischen Fette insbesondere Trane, Fischöl, Rindertalg und Rinderklauenöl, aus der Gruppe der pflanzlichen Fette Rizinusöl, Rüböl und Leinöl herangezogen. In Wachsen und Harzen sind die Fettsäuren statt mit Glycerin mit höhermolekularen Alkoholen verestert. Beispiele für Wachse sind Bienenwachs, chinesisches Wachs, Carnaubawachs, Montanwachs und Wollfett; zu den wichtigsten Harzen zählen Kolophonium, Juchtenöl und Schellack.
- Durch chemische Umwandlung pflanzlicher und tierischer Fette erhält man Produkte, die wasserlöslich sind und die darüber hinaus in unterschiedlichem Maße emulgierend auf wasserunlösliche Fettstoffe wirken. Bekannt sind etwa die sulfierten wasserlöslichen Öle verschiedenster Art, die durch Oxidation veränderten Trane, die als Dégras oder Moellon bezeichnet werden, ferner die Seifen, die bei der hydrolytischen Spaltung natürlicher Fette entstehen, gehärtete Fette sowie schließlich freie Fettsäuren wie Stearinsäure als Einbrennfette. Die meisten tierischen und pflanzlichen Fette weisen eine gewisse Affinität zur Ledersubstanz auf, die durch die Einführung oder Freilegung hydrophiler Gruppen noch beträchtlich gesteigert wird.
- Wichtig für die Lederherstellung sind weiter die mineralischen Fettungsmittel. Diese Kohlenwasserstoffe sind den natürlichen Fetten und Ölen in manchen Eigenschaften ähnlich, lassen sich jedoch nicht verseifen. Es handelt sich um Fraktionen der Erdöldestillation, die in flüssiger Form Mineralöl, in pastöser Form Vaseline und in fester Form Paraffin genannt werden.

Üblicherweise werden zur Lederfettung anionische Tenside eingesetzt. So sind beispielsweise aus der europäischen Patentschrift **EP-B 0247509** (Stockhausen) Anlagerungsprodukte von Schwefelsäure bzw. Oleum an ungesättigte, alkoxylierte sowie gegebenenfalls epoxidierte Fette und Öle bekannt, Hierbei ist jedoch von Nachteil, daß nur ein geringer Anteil der Schwefelsäure bzw. des Oleums wirklich zur Sulfatierung ausgenutzt werden kann, während das nicht abreagierte Sulfatierungsmittel anschließend neutralisiert werden muß. Auf diese Weise wird das Wertprodukt mit einer ganz erheblichen Menge an Elektrolytsalzen belastet, die nicht nur das Verfahren verteuern, sondern im Gegenteil absolut unerwünscht sind, weil sie die anwendungstechnischen Eigenschaften der Mittel nachteilig beeinflussen und die Korrosivität erhöhen. Bislang ist es daher erforderlich, die Sulfatierungsprodukte mit erheblichem Aufwand nachträglich zu entsalzen, um den Elektrolytgehalt wenigstens auf Werte von 4 bis 5 Gew.-% zu begrenzen.

Die Aufgabe der Erfindung hat somit darin bestanden, sulfatierte Fettsäureester zur Verfügung zu stellen, die gleichzeitig einen höheren Gehalt an Aktivsubstanz und einen verminderten Elektrolytgehalt aufweisen, ohne daß hierzu eine separate Salzabtrennung erforderlich ist.

EP 353 704 A1 betrifft flüssige bzw. fließfähige Derivate von natürlichen Fetten und Ölen und ein Verfahren zur Herstellung dieser Derivate, bei dem bei erhöhten Temperaturen in Gegenwart basischer Katalysatoren mit wenigstens einer Epoxidgruppe enthaltenden Verbindung oxalkyliert und auf an sich bekannte Weise sulfiert wird. Als Ausgangsmaterial werden dabei bestimmte Fettsäureester aliphatischer Monoalkohole oder deren Mischung mit bei Raumtemperatur festen bzw. feste Anteile enthaltenen Fetten eingesetzt.

### Beschreibung der Erfindung

Gegenstand der Erfindung ist ein Verfahren zur Herstellung von sulfatierten Fettsäureestem mit vermindertem Elektrolytgehalt, bei dem man
(a) Ester von ungesättigten Fettsäuren mit niederen Alkoholen mit 1 bis 4 Kohlenstoffatomen oder Glycerin mit Schwefelsäure oder Oleum sulfatiert,
(b) nicht umgesetztes Sulfatierungsmittel durch Ringöffnung von gegebenenfalls estergruppensubstituierten Epoxyalkanen ganz oder teilweise in organisch gebundenes Sulfat überführt, und
(c) die Reaktionsprodukte durch Zugabe von Basen auf einen alkalischen pH-Wert einstellt.

Im Gegensatz zu Verfahren des Stands der Technik, bei denen in der Sulfatierung nicht umgesetzte Schwefelsäure bzw. Oleum nach der Neutralisation ausgewaschen werden muß, können nach dem erfindungsgemäßen Verfahren Produkte mit höherem Aktivsubstanzgehalt erhalten werden, da das restliche ungenutzte Sulfatierungsmittel durch Umsetzung mit den Epoxyalkanen in Wertsubstanz überführt wird. Durch diese Maßnahme wird gleichzeitig die Menge an freier Schwefelsäure vermindert, so daß weniger Neutralisationsbase verbraucht und der Elektrolytgehalt im Endprodukt verringert wird.

### Ungesättigte Fettsäureester

Die ungesättigten Fettsäureester, die im Sinne der Erfindung als Ausgangsstoffe in Betracht kommen, stellen vorzugsweise Ester von Fettsäuren der Formal **(I)** dar,

**R**^{**1**}**COOH (I)**

in der R¹CO für einen ungesättigten Acylrest mit 12 bis 24 Kohlenstoffatomen und 1 bis 5 Doppelbindungen steht. Typische Beispiele hierfür sind Palmoleinsäure, Ölsäure, Elaidinsäure, Petroselinsäure, Linolsäure, Linolensäure, Ricinolsäure, Gadoleinsäure und Erucasäure sowie deren technische Mischungen, die herstellungsbedingt noch in untergeordneten Mengen (d.h. weniger als 50 Gew.-%) gesättigte Anteile aufweisen können. Die Alkoholgruppe kann sich von niederen Alkoholen mit 1 bis 4 Köhlenstoffatomen oder Glycerin ableiten. Demzufolge können die Einsatzstoffe beispielsweise ungesättigte Fettsäuremethylester, ungesättigte Fettsäurepartialglyceride (Mono- und/oder Diglyceride) und/oder ungesättigte Fettsäuretriglyceride darstellen. Letztere können dabei synthetischer oder natürlicher Herkunft sein. So kann man beispielsweise Glycerintrioleat einsetzen oder ein ungesättigtes Öl wie beispielsweise Olivenöl, Sonnenblumenöl, Rapsöl, Erdnußöl, Leinöl, Rindertalg, Fischöl und dergleichen. Die ungesättigten Ester, insbesondere die ungesättigten Triglyceride, besitzen lodzahlen im Bereich von 50 bis 150, vorzugsweise 70 bis 100.

### Sulfatierung

Die Sulfatierung kann in an sich bekannter Weise durchgeführt werden, d.h. die ungesättigten Fettsäureester werden portionsweise mit Schwefelsäure oder Oleum versetzt, wobei es dann zur Anlagerung von H₂SO₄ an die Doppelbindung kommt. Üblicherweise werden die ungesättigten Fettsäureester und die Sulfatierungsmittel im molaren Verhältnis von 1 : 0,3 bis 1 : 1, vorzugsweise 0,5 bis 0,8 - bezogen auf die Doppelbindungsäquivalente - eingesetzt, wobei die Reaktionstemperatur unter 40°C gehalten wird.

### Epoxyalkane

Der zweite Schritt des Verfahrens besteht darin, die nicht umgesetzte Menge an Sulfatierungsmittel zur Ringöffnung von Epoxyalkanen auszunützen. Der Vorteil dieses Verfahrensschrittes besteht einmal darin, daß Schwefelsäure nicht durch Neutralisation in unerwünschtes anorganisches Sulfat überführt sondern umgekehrt genutzt wird, durch Ringöffnung des Epoxyalkans wertvolles Aniontensid herzustellen, welches mit dem sulfatierten Fettsäureester zudem noch eine synergistische Leistungssteigerung in der Lederfettung ergibt. Die Erfindung schließt die Erkenntnis ein, daß die Ringöffnungsreaktion spontan und vollständig abläuft. Üblicherweise setzt man end- oder innenständige Epoxyalkane der Formel **(II)** ein, 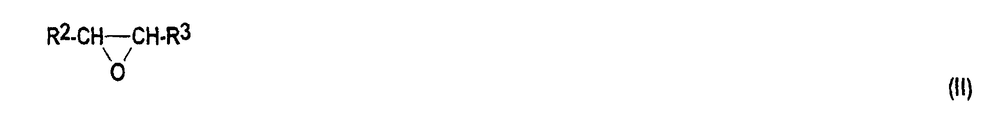 in der R² und R³ unabhängig voneinander für Wasserstoff oder Alkylreste mit 1 bis 16 Kohlenstoffatomen oder einen Rest der Formel **(III)** stehen können,

**-(CH2)**_{**n**}**COOR**^{**4**} **(II)**

in der R⁴ einen Alkylrest mit 1 bis 4 Kohlenstoffatomen darstellt und n eine Zahl im Bereich von 1 bis 10 bedeutet, mit der Maßgabe, daß die Summe der Kohlenstoffatome in den beiden Resten R² und R³ im Bereich von 10 bis 16 liegt. Typische Beispiele sind innenständige Epoxyalkane wie 2,3-, 3,4-, und 5,6-Epoxydodecan, 6,7-Epoxytetradecan, 7,8-Epoxyhexadecan und 8,9-Epoxyoctadecan sowie die bevorzugten 1,2-Epoxyalkane wie beispielsweise 1,2-Epoxydodecan, 1,2-Epoxytetradecan, 1,2-Epoxyhexadecan und 1,2-Epoxyoctadecan. Des weiteren kommen epoxidierte Fettsäureniedrigalkylester, vorzugsweise epoxidierte Fettsäuremethylester in Betracht, bei denen sich die Acylgruppe von ungesättigten Fettsäuren mit 16 bis 22 Kohlenstoffatomen ableiten kann. Typische Beispiele sind epoxidierte Methylester der Ölsäure, Elaidinsäure, Petroselinsäure, Linolsäure, Linolensäure, Konjuenfettsäure, Ricinolsäure, Gadoleinsäure und Erucasäure sowie deren technische Mischungen.

### Ringöffnung

Die Ringöffnung kann ebenfalls in an sich bekannter Weise durchgeführt werden, wobei man die Epoxyalkane bezogen auf die Menge an nicht umgesetztem Sulfatierungsmittel in Mengen von 50 bis 300, vorzugsweise 100 bis 200 und insbesondere 110 bis 120 Mol-% einsetzt. Es empfiehlt sich, das Epoxid portionsweise zuzugeben, so daß die exotherme Reaktion 70°C nicht übersteigt.

### Neutralisation

In Abhängigkeit von der eingesetzten Menge Epoxyalkan ist im Reaktionsgemisch noch freie Schwefelsäure enthalten, die durch Zugabe von gegebenenfalls wäßrigen Basen neutralisiert werden muß, wobei man die Produkte vorzugsweise auf einen pH-Wert im Bereich von 8 bis 10 einstellt. Als wäßrige Basen kommen beispielsweise 5 bis 55, vorzugsweise 25 bis 35 Gew.-%ige Lösungen von Natriumhydroxid, Kaliumhydroxid, Calciumoxid oder Ammoniak in Frage. Die Neutralisation kann jedoch auch wasserfrei mit Alkylaminen oder Alkanolaminen wie beispielsweise Methylamin, Dimethylamin, Monoethanolamin, Diethanolamin und Triethanolamin durchgeführt werden.

### Cotenside

Die erfindungsgemäßen Sulfierungsprodukte können alleine, vorzugsweise jedoch in Abmischung mit anderen anionischen, nichtionischen, amphoteren bzw. zwitterionischen Tensiden und - eingeschränkt - auch kationischen Tensiden eingesetzt werden. Typische Beispiele für **anionische Tenside** sind Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, Acyllactylate, Alkyloligoglucosidsulfate und Alkyl(ether)phosphate. Sofern die anionischen Tenside Polyglycoletherketten enthalten, können sie eine konventionelle, vorzugsweise jedoch eine eingeengte Homologenverteilung aufweisen. Typische Beispiele für **nichtionische Tenside** sind Fettalkoholpolyglycolether, Alkylphenolpolyglycolether, Fettsäurepolyglycolester, Fettsäureamidpolygylcolether, Fettaminpolyglycolether, alkoxylierte Triglyceride, Alk(en)yloligoglykoside, Fettsäure-N-alkylglucamide, Polyolfettsäureester, Zuckerester, Sorbitanester und Polysorbate. Sofern die nichtionischen Tenside Polyglycoletherketten enthalten, können sie eine konventionelle, vorzugsweise jedoch eine eingeengte Homologenverteilung aufweisen. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Typische Beispiele für **kationische Tenside**, mit denen die anionischen Sulfierungsprodukte verträglich sind und keine schwerlöslichen Salze bilden, sind Esterquats, insbesondere solche, die über Ethylenoxideinheiten im Molekül verfügen. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S.54-124** oder **J.Falbe (ed.), "Katalysatoren, Tenside und Mineralöladditive", Thieme Verlag, Stuttgart, 1978, S.123-217** verwiesen.

### Gewerbliche Anwendbarkeit

Die Reaktionsprodukte weisen ein ausgezeichnetes Fettungsverhalten für Leder und Pelze auf. Ein weiterer Gegenstand der Erfindung betrifft daher die Verwendung der nach dem erfindungsgemäßen Verfahren erhältlichen Sulfatierungsprodukte zur Herstellung von Mitteln zur Behandlung von Ledern und Pelzen, insbesondere Lederfettungsmitteln. Die Zubereitungen können neben den Sulfatierungspro-dukten und anderen Tensiden weitere gebräuchliche Hilfs- und Zusatzstoffe enthalten. Üblicherweise beträgt der Anteil der Sulfatierungsprodukte an den Mitteln 15 bis 90 und vorzugsweise 20 bis 80 Gew.-% bezogen auf die Zubereitungen. In der Regel werden die Mittel so dosiert, daß auf 1 kg Leder bzw. Pelz (berechnet als Falzgewicht) 20 bis 1000, vorzugsweise 30 bis 80 g des Mittels entfallen.

### Beispiele

**Beispiel 1.** In einem 1-I-Dreihalskolben mit Rührer, Rückflußkühler und Tropftrichter wurden 500 g (0,57 Mol) Glycerintrioleat vorgelegt. innerhalb von 2 h wurden 89 g (0,91 Mol) konzentrierte Schwefelsäure unter starkem Rühren mit einer solchen Geschwindigkeit zugetropft, daß die Temperatur nicht über 35°C anstieg. Anschließend wurde die Reaktionsmischung 2 weitere Stunden bei 30°C gerührt, wobei der Gehalt an freier Schwefelsäure anschließend noch 6,7 Gew.-% betrug. Danach wurden 95 g (0,52 Mol) 1,2-Epoxydodecan zugetropft und der Ansatz weitere 2 h gerührt. Die resultierende weiterhin saure Mischung wurde anschließend in eine Lösung von 108 g Ammoniak in 121 g Wasser gegeben und neutralisiert, wobei die Temperatur unter 70°C gehalten wurde. Das Produkt wurde als klare Flüssigkeit erhalten, der Ammoniumsulfatgehalt betrug 2,8 Gew.-%.

**Beispiel 2.** Analog Beispiel 1 wurden zu 589 g eines Umsetzungsproduktes von Glycerintrioleat mit Schwefelsäure mit einem Gehalt an freier H₂SO₄ von 7 Gew.-% 209 g (1,1 Mol) 1,2-Epoxydodecan gegeben. Es wurde ein Produkt mit einem Ammoniumsulfatgehalt von 1,9 Gew.-% erhalten.

**Beispiel 3 : Herstellung von Schafbekleidungsleder.** Wet blue-Material wurde zunächst gewaschen und im Anschluß nachgegerbt. Die Angaben zu Einsatzmengen und Zeitdauer der Verfahrensschritte sind in Tabelle 1 zusammengefaßt. Alle Prozentangaben verstehen sich bezogen auf das Falzgewicht.

**Tabelle 1**

| **Herstellung von Schafbekleidungsleder (Mengenangaben als Gew.·%)** | | | |
|---|---|---|---|
| **Vorgang** | **Zugabe von** | **Menge [%]** | **Dauer [min]** |
| Waschen | Wasser (40°C) | 200 | 10 |
| Vorbehandlung | Wasser (40°C) | 100 | 40 |
| | Natriumformiat | 1 | |
| | Natriumhydrogencarbonat | 1 | |
| | Acrylatgerbstoff | 3 | |
| | Flotte ausspülen (50°C) | | |
| Waschen | Wasser (50°C) | 100 | 30 |
| Färben | Naphthalinkondensationsprodukt | 2 | 30 |
| | Farbstoff | 3 | |
| Fetten | Lecithinemulsion | 3 | 40 |
| | Sulfatiertes Glycerintrioleat/ | 5 | 40 |
| | Hydroxydodecansulfat (Bsp. 1) | | |
| Gerben | Acrylatgerbstoff | 2 | 60 |
| Nachbehandlung | Ameisensäure | 1 | 15 |
| | Flotte ausspülen (50°C) | | |

## Patentansprüche

1. Verfahren zur Herstellung von sulfatierten Fettsäureestem mit vermindertem Elektrolytgehalt, bei dem man
(a) Ester von ungesättigten Fettsäuren mit niederen Alkoholen mit 1 bis 4 Kohlenstoffatomen oder Glycerin mit Schwefelsäure oder Oleum sulfatiert,
(b) nicht umgesetztes Sulfatierungsmittel durch Ringöffnung von gegebenenfalls estergruppensubstituierten Epoxyalkanen ganz oder teilweise in organisch gebundenes Sulfat überführt, und
(c) die Reaktionsprodukte durch Zugabe von Basen auf einen alkalischen pH-Wert einstellt.

2. Verfahren nach Anspruch 1, **dadurch gekennzeichnet, daß** man Ester von Fettsäuren der Formel **(I)** einsetzt,
**R**^{**1**}**COOH (I)**
in der R¹CO für einen ungesättigten Acylrest mit 12 bis 24 Kohlenstoffatomen und 1 bis 5 Doppelbindungen steht.

3. Verfahren nach den Ansprüchen 1 und 2, **dadurch gekennzeichnet, daß** man ungesättigte Fettsäuremethylester, Fettsäurepartialglyceride und oder Fettsäuretriglyceride einsetzt.

4. Verfahren nach den Ansprüchen 1 bis 3, **dadurch gekennzeichnet, daß** man ungesättigte Ester mit einer lodzahl im Bereich von 50 bis 150 einsetzt.

5. Verfahren nach den Ansprüchen 1 bis 4, **dadurch gekennzeichnet, daß** man die ungesättigten Fettsäureester und die Sulfatierungsmittel im molaren Verhältnis von 1 : 0,3 bis 1 : 1 - bezogen auf die Doppelbindungsäquivalente - einsetzt.

6. Verfahren nach den Ansprüchen 1 bis 5, **dadurch gekennzeichnet, daß** man end- oder innenständige Epoxyalkane der Formel **(II)** einsetzt, 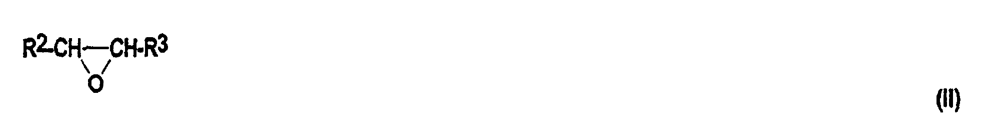 in der R² und R³ unabhängig voneinander für Wasserstoff oder Alkylreste mit 1 bis 16 Kohlenstoffatomen oder einen Rest der Formel **(III)** stehen können,
**-(CH2)**_{**n**}**COOR**^{**4**} **(III)**
in der R⁴ einen Alkylenrest mit 1 bis 4 Kohlenstoffatomen darstellt und n eine Zahl im Bereich von 1 bis 10 bedeutet, mit der Maßgabe, daß die Summe der Kohlenstoffatome in den beiden Resten R² und R³ im Bereich von 10 bis 16 liegt.

7. Verfahren nach den Ansprüchen 1 bis 6, **dadurch gekennzeichnet, daß** man 1,2-Epoxyalkane und/oder epoxidierte Fettsäuremethylester einsetzt.

8. Verfahren nach den Ansprüchen 1 bis 7, **dadurch gekennzeichnet, daß** man die Epoxyalkane bezogen auf die Menge an nicht umgesetzten Sulfatierungsmittel in Mengen von 50 bis 300 Mol-% einsetzt.

9. Verfahren nach den Ansprüchen 1 bis 8, **dadurch gekennzeichnet, daß** man die Reaktionsprodukte durch Zugabe von Basen auf einen pH-Wert im Bereich von 8 bis 10 einstellt.

10. Verwendung der Reaktionsprodukte nach dem Verfahren nach den Ansprüchen 1 bis 9 zur Herstellung von Mitteln zur Behandlung von Ledem und Pelzen.

## Claims

1. A process for the production of sulfated fatty acid esters with a reduced electrolyte content, in which
(a) esters of unsaturated fatty acids with lower C₁₋₄ alcohols or glycerol are sulfated with sulfuric acid or oleum,
(b) unreacted sulfating agent is partly or completely converted into organically bound sulfate by ring opening of optionally ester-group-substituted epoxyalkanes and
(c) the reaction products are adjusted to an alkaline pH by addition of bases.

2. A process as claimed in claim 1, **characterized in that** esters of fatty acids corresponding to formula (I):
R¹COOH (I)
in which R¹CO is an unsaturated acyl group containing 12 to 24 carbon atoms and 1 to 5 double bonds,
are used.

3. A process as claimed in claims 1 and 2, **characterized in that** unsaturated fatty acid methyl esters, fatty acid partial glycerides and/or fatty acid triglycerides are used.

4. A process as claimed in claims 1 to 3, **characterized in that** unsaturated esters with an iodine value of 50 to 150 are used.

5. A process as claimed in claims 1 to 4, **characterized in that** the unsaturated fatty acid esters and the sulfating agents are used in a molar ratio of 1:0.3 to 1:1, based on the double bond equivalents.

6. A process as claimed in claims 1 to 5, **characterized in that** terminal or internal epoxyalkanes corresponding to formula (II): 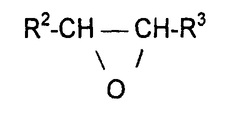 in which R² and R³ independently of one another may represent hydrogen or C₁₋₁₆ alkyl groups or a group of the following formula:
-(CH₂)ₙCOOR⁴ (III)
in which R⁴ is an alkylene group containing 1 to 4 carbon atoms and n is a number of 1 to 10, with the proviso that the sum of the carbon atoms in the two substituents R² and R³ is in the range from 10 to 16,
are used.

7. A process as claimed in claims 1 to 6, **characterized in that** 1,2-epoxyalkanes and/or epoxidized fatty acid methyl esters are used.

8. A process as claimed in claims 1 to 7, **characterized in that** the epoxyalkanes are used in quantities of 50 to 300 mol-%, based on the quantity of unreacted sulfating agent.

9. A process as claimed in claims 1 to 8, **characterized in that** the reaction products are adjusted to a pH of 8 to 10 by addition of bases.

10. The use of the reaction products obtained by the process claimed in claims 1 to 9 for the production of preparations for treating leathers and pelts.

## Revendications

1. Procédé de préparation d'esters d'acide gras sulfatés ayant une teneur réduite en électrolytes dans lequel ;
a) des esters d'acide gras non saturé avec des alcools inférieurs ayant de 1 à 4 atomes de carbone ou avec du glycérol, sont sulfatés à l'acide sulfurique ou l'oléum,
b) un agent de sulfatation qui n'a pas réagi est converti totalement ou partiellement en sulfate lié organiquement par ouverture du cycle d'époxyalkanes éventuellement substitués par des groupes ester, et
c) les produits de la réaction sont ajustés à un pH alcalin par addition de bases.

2. Procédé selon la revendication 1,
**caractérisé en ce qu'**
on met en oeuvre des esters d'acide gras de formule (I) :
**R**^{**1**} **COOH (I)**
dans laquelle R¹ CO représente un reste acyle non saturé ayant de 12 à 24 atomes de carbone et de 1 à 5 doubles liaisons.

3. Procédé selon les revendications 1 et 2,
**caractérisé en ce qu'**
on met en oeuvre un ester méthylique d'acide gras non saturé, des glycérides partiels d'acide gras et/ou des triglycérides d'acide gras.

4. Procédé selon les revendications 1 à 3,
**caractérisé en ce qu'**
on met en oeuvre des esters non saturés ayant un indice d'iode dans la zone de 50 à 150.

5. Procédé selon les revendications 1 à 4,
**caractérisé en ce qu'**
on met en oeuvre les esters d'acide gras non saturé et l'agent de sulfatation dans un rapport molaire de 1 : 0,3 à 1 : 1 rapporté aux équivalents en double liaison.

6. Procédé selon les revendications 1 à 5,
**caractérisé en ce qu'**
on met en oeuvre des époxyalkanes terminaux ou situés à l'intérieur de la formule II 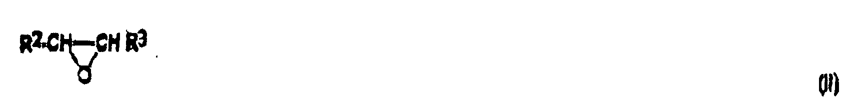 dans laquelle R² et R³ indépendamment l'un de l'autre peuvent représenter de l'hydrogène ou des restes allyle ayant de 1 à 16 atomes de carbone ou un reste de formule III :
**(CH**_{**2**}**)**_{**n**} **COOR**^{**4**} **(III)**
dans laquelle R⁴ représente un reste alkylène ayant de 1 à 4 atomes de carbone et n signifie un nombre dans la zone de 1 à 10 avec la précision que la somme des atomes de carbone dans les deux restes R² et R³ se situe dans la zone de 10 à 16.

7. Procédé selon les revendications 1 à 6,
**caractérisé en ce qu'**
on met en oeuvre des 1,2 - époxyalkanes et/ou des esters méthyliques d'acide gras époxydé.

8. Procédé selon les revendications 1 à 7,
**caractérisé en ce qu'**
on met en oeuvre les époxy alkanes rapporté à la quantité en agent de sulfatation qui n'a pas réagi, en quantités allant de 50 à 300 % molaire.

9. Procédé selon les revendications 1 à 8,
**caractérisé en ce qu'**
on ajuste les produits de la réaction à une valeur de pH dans la zone de 8 à 10.

10. Utilisation des produits de réaction selon le procédé selon les revendications 1 à 9, en vue de la production d'agent pour le traitement des cuirs et des peaux.
